# EUROPÄISCHE PATENTSCHRIFT

(11) **EP 3 680 224 B1**
(45) Veröffentlichungstag und Bekanntmachung des Hinweises auf die Patenterteilung: **24.05.2023**
(21) Anmeldenummer: 19150659.1
(22) Anmeldetag: 08.01.2019
(51) Int. Cl.: C07C 7/163, C07C 11/08, C07C 11/10, C10G 45/40, C10G 65/06

(54) **VERFAHREN ZUR ENTFERNUNG MEHRFACH UNGESÄTTIGTER KOHLENWASSERSTOFFE AUS C4-KOHLENWASSERSTOFFSTRÖMEN IN ANWESENHEIT VON MERCAPTANEN, DISULFIDEN UND C5-KOHLENWASSERSTOFFEN**
PROCESS FOR REMOVING MULTIPLY UNSATURATED HYDROCARBONS FROM C4 HYDROCARBON FLOWS IN THE PRESENCE OF MERCAPTAN, DISULFIDES AND C5 HYDROCARBONS
PROCÉDÉ D'ÉLIMINATION DES HYDROCARBURES PLUSIEURS FOIS INSATURÉS DU FLUX D'HYDROCARBURES EN C4 EN PRÉSENCE DES MERCAPTANS, DES DISULFURES ET DES HYDROCARBURES EN C5

(43) Veröffentlichungstag der Anmeldung: 15.07.2020
(73) Patentinhaber: Evonik Operations GmbH, 45128 Essen (DE)
(72) Erfinder: PEITZ, Stephan, 45739 Oer-Erkenschwick (DE); STOCHNIOL, Guido, 45721 Haltern am See (DE); WINTERBERG, Markus, 45731 Waltrop (DE); SCHALLENBERG, Jörg, 46286 Marl (DE); RIX, Armin Matthias, 45770 Marl (DE); WOLFF, Andreas, 46657 Recklinghausen (DE)
(74) Vertreter: Evonik Patent Association

(56) Entgegenhaltungen:
- WO-A1-2012/004081
- WO-A1-2014/209736

## Beschreibung

Die Erfindung betrifft ein zweistufiges Verfahren zur Entfernung von mehrfach ungesättigten Kohlenwasserstoffen aus C4-Kohlenwasserstoffströmen, welche neben C4-Kohlenwasserstoffen auch C5-Kohlenwasserstoffe und Mercaptane und/oder Disulfide enthalten.

In der petrochemischen Industrie eingesetzte olefinhaltige C4-Kohlenwasserstoffströme sind typischerweise C4-Kohlenwasserstoffgemische aus katalytischen Crackern (FCC-C4) oder aus Steamcrackern (Crack-C4). Auch Mischungen von C4-Kohlenwasserstoffgemischen unterschiedlicher Herkunft (C4-Schnitt) können in petrochemischen Verfahren eingesetzt werden. Technische C4-Kohlenwasserstoffgemische, wie die vorgenannten Gemische, enthalten typischerweise neben den n-Butenen 1-Buten, cis-2-Buten und trans-2-Buten, Isobuten und den homologen Alkanen Butan und Isobutan auch mehrfach ungesättigte Verbindungen, insbesondere 1 ,3-Butadien, But-3-en-1-in und 1 ,2-Butadien. Bevor die C4-Kohlenwasserstoffgemische für Prozesse wie die Oligomerisierung zur Verfügung stehen oder bevor einzelne Verbindungen aus den C4-Gemischen isoliert werden können, ist häufig die Entfernung der mehrfach ungesättigten Kohlenwasserstoffe notwendig.

C4-Kohlenwasserstoffgemische aus Steamcrackern oder katalytischen Crackern können hohe Anteile an mehrfach ungesättigten Verbindungen, insbesondere hohe Anteile (40+ Gew.-%) an 1,3-Butadien enthalten. Die Aufarbeitung erfolgt dann zunächst so, dass die Menge der mehrfach ungesättigten Verbindungen, beispielsweise mittels einer Extraktivdestillation, auf Werte von kleiner oder gleich 1 Gew.-% bezogen auf die Gesamtzusammensetzung des C4-Kohlenwasserstoffgemisches abgesenkt wird. Das resultierende Gemisch mit nur noch geringen Anteilen an Butadien wird auch als Raffinat I bezeichnet, aus dem dann Isobuten zur Herstellung von Raffinat II entfernt werden kann.

Die weitere Aufarbeitung umfasst dann typischerweise einen weiteren Schritt zur (nahezu) vollständigen Entfernung der mehrfach ungesättigten Kohlenwasserstoffe. Die EP 2 590 913 A1 offenbart dazu eine Selektivhydrierung der noch vorhandenen mehrfach ungesättigten Kohlenwasserstoffe in Anwesenheit von Wasserstoff. Dabei muss zur Vermeidung von unerwünschten Nebenreaktionen und Anlagenzuständen der benötigte Wasserstoff im C4-Zulaufgemisch homogen gelöst vorliegen. Die Löslichkeit von Wasserstoff ist in C4-Kohlenwasserstoffgemischen jedoch nach oben begrenzt, was eine Beschränkung des maximal im Zulauf zur Selektivhydrierung zulässigen Gehaltes an mehrfach ungesättigten Kohlenwasserstoff ergibt, nämlich genau die Menge an mehrfach ungesättigten Kohlenwasserstoffen, die durch die maximal lösliche Menge an Wasserstoff (selektiv) hydriert werden kann.

Um einen (nahezu) vollständigen Umsatz der mehrfach ungesättigten Kohlenwasserstoffe zu erreichen, dürfen die eingesetzten C4-Kohlenwasserstoffgemische nur die maximal Menge an mehrfach ungesättigten Kohlenwasserstoffen enthalten, die mit der maximal löslichen Menge an Wasserstoff hydriert werden kann. Das ist für technische Prozesse insofern problematisch, als das die Zusammensetzung der eingesetzten C4-Kohlenwasserstoffgemische natürlichen Schwankungen unterliegt, die mit einer solchen Limitierung in Bezug auf die maximale Menge an mehrfach ungesättigten Kohlenwasserstoffen nur schwer abgefangen können.

Die Aufgabe der vorliegenden Erfindung bestand deshalb darin ein Verfahren bereitzustellen, welches flexibler auf Schwankungen in der Menge an mehrfach ungesättigten Kohlenwasserstoffen reagieren kann und womit potentiell größere Mengen an mehrfach ungesättigten Kohlenwasserstoffen aus den C4-Kohlenwasserstoffströmen entfernt werden können.

Gelöst wird die zugrundeliegende Aufgabe der vorliegenden Erfindung durch das Verfahren gemäß Anspruch 1. Bevorzugte Ausführungsformen sind in den Unteransprüchen angegeben.

Das erfindungsgemäße Verfahren ist ein zweistufiges Verfahren zur Entfernung von mehrfach ungesättigten Kohlenwasserstoffen aus olefinhaltigen C4-Kohlenwasserstoffströmen, welche neben C4-Kohlenwasserstoffen auch C5-Kohlenwasserstoffe im Bereich zwischen 0,01 und 5 Gew.-% und Mercaptane und/oder Disulfide im Bereich zwischen 0,01 und 200 Gew.-ppm enthalten. Dabei wird in dem ersten Schritt zumindest ein Teil der mehrfach ungesättigten Kohlenwasserstoffe aus dem olefinhaltigen C4-Kohlenwasserstoffstrom in einer ersten Reaktionszone, die aus einem oder mehreren parallel und/oder in Reihe geschalteten Reaktoren besteht, hydriert und/oder mit den Mercaptanen und/oder Disulfiden aus dem olefinhaltigen C4-Kohlenwasserstoffstrom umgesetzt, wobei der erste Schritt unter Verwendung eines ersten Katalysators, der mindestens zwei Metalle der 10. Gruppe des Periodensystems der Elemente, vorzugsweise Palladium und Platin, enthält, in Anwesenheit von Wasserstoff und in Abwesenheit von Kohlenmonoxid durchgeführt wird und wobei in dem ersten Schritt maximal 80 Mol-% der vorhandenen mehrfach ungesättigten Kohlenwasserstoffe aus dem C4-Kohlenwasserstoffstrom hydriert und/oder umgesetzt werden, wobei das molare Verhältnis von Wasserstoff zu mehrfach ungesättigten Kohlenwasserstoffen zwischen 0,01 und 0,8 beträgt. In einer bevorzugten Ausführungsform werden die mehrfach ungesättigten Kohlenwasserstoffen teilweise hydriert und teilweise mit den Mercaptanen und/oder Disulfiden aus dem olefinhaltigen C4-Kohlenwasserstoffstrom umgesetzt. Auch dabei wird die maximal umzusetzende Menge von 80 Mol-% an mehrfach ungesättigten Kohlenwasserstoffen nicht überschritten. Vorzugsweise werden die Mercaptane bis unter die Nachweisgrenze aus dem olefinhaltigen C4-Kohlenwasserstoffstrom entfernt.

Nach dem ersten Schritt und vor dem zweiten Schritt erfolgt mindestens eine Schwersiederabtrennung, wobei insbesondere die bei der Reaktion der mehrfach ungesättigten Kohlenwasserstoffe mit den Mercaptanen und/oder Disulfiden gebildeten Umsetzungsprodukte und optional zumindest ein Teil der C5-Kohlenwasserstoffe aus dem C4-Kohlenwasserstoffstrom entfernt werden.

Im zweiten Schritt des erfindungsgemäßen Verfahrens werden die im ersten Schritt nicht umgesetzten mehrfach ungesättigten Kohlenwasserstoffe in dem olefinhaltigen C4-Kohlenwasserstoffstrom in einer zweiten Reaktionszone, die aus einem oder mehreren parallel und/oder in Reihe geschalteten Reaktoren besteht, unter Verwendung eines zweiten Katalysators, der ein einziges Metall der 10. Gruppe des Periodensystems der Elemente, vorzugsweise Palladium, enthält, in Anwesenheit von Wasserstoff und Kohlenmonoxid selektiv hydriert. Der im ersten Schritt eingesetzte Katalysator und der im zweiten Schritt eingesetzte Katalysator sind dabei unterschiedlich voneinander.

Der erfindungsgemäße Verfahren hat den Vorteil, dass im ersten Schritt nicht nur zumindest ein Teil der mehrfach ungesättigten Kohlenwasserstoffe, insbesondere 1,3-Butadien und/oder 1,2-Butadien und/oder But-3-en-1 hydriert werden können, sondern auch Mercaptane und/oder Disulfide, die in technischen C4-Kohlenwasserstoffgemischen in Kleinstmengen vorhanden sind und aufgrund ihrer für nachfolgende Prozesse katalysatorschädigenden Eigenschaften entfernt werden müssen, umgesetzt und dann abgetrennt werden können. Die maximale zulässige Menge an mehrfach ungesättigten Kohlenwasserstoffen im aufzureinigenden olefinhaltigen C4-Kohlenwasserstoffstrom wird dadurch im Vergleich zur Selektivhydrierung fast verdoppelt. Die Konzentration von mehrfach ungesättigten Kohlenwasserstoffen in technischen C4-Kohlenwasserstoffströmen unterliegt prozessbedingten Schwankungen. Diese Schwankungen in der Konzentration an mehrfach ungesättigten Kohlenwasserstoffen in den technischen C4-Kohlenwasserstoffstrom können somit durch das erfindungsgemäße Verfahren viel einfacher abgefangen werden.

Der erste Schritt, also die Hydrierung der mehrfach ungesättigten Kohlenwasserstoffe und/oder die Umsetzung der mehrfach ungesättigten Kohlenwasserstoffe mit den Mercaptanen und/oder Disulfiden im C4-Kohlenwasserstoffstrom, erfolgt in der Anwesenheit von Wasserstoff. Um zu verhindern, dass Nebenreaktionen wie die Vollhydrierung zu Alkanen oder eine Isomerisierung der Doppelbindung von 1-Olefinen zu internen Olefinen auftreten, sollte die Wasserstoffmenge, die im ersten Schritt eingesetzt wird, nicht zu hoch sein. Das molare Verhältnis von Wasserstoff zu mehrfach ungesättigten Kohlenwasserstoffen im ersten Schritt liegt zwischen 0,01 und 0,8, vorzugsweise beträgt es zwischen 0,1 und 0,5. Der Wasserstoff kann dabei separat zum einströmenden C4-Kohlenwasserstoffstrom in die Reaktionszone eindosiert werden oder dem einströmenden C4-Kohlenwasserstoffstrom vorher hinzugefügt werden, wobei im Reaktor ein homogenes Gemisch aus C4-Kohlenwasserstoffstrom und Wasserstoff vorliegen muss.

Der erste Schritt des erfindungsgemäßen Verfahrens wird zudem in Abwesenheit von Kohlenmonoxid durchgeführt. Das heißt, dass dem C4-Kohlenstoffstrom kein Kohlenmonoxid hinzugefügt wird. Kleinstmengen an Kohlenmonoxid, die sich möglicherweise als Verunreinigungen in technischen Kohlenwasserstoffströmen vorhanden sein können, können jedoch vorhanden sein, da sie nicht ohne erheblichen Aufwand entfernt werden können.

Als erster Katalysator im ersten Schritt des erfindungsgemäßen Verfahrens werden heterogene Katalysatoren eingesetzt, die mindestens zwei Metalle der 10. Gruppe des Periodensystems der Elemente enthalten, vorzugsweise Palladium und Platin. Die Metalle können aufgrund von Kalzination partiell in Form ihres Oxids vorliegen. Vorzugsweise liegen die Metalle auf einem Trägermaterial vor. Das Trägermaterial ist ausgewählt aus der Gruppe, bestehend aus Aluminiumoxid, Kieselgel und Aktivkohle, wobei bevorzugt Aluminiumoxid als Trägermaterial eingesetzt wird. In einer besonders bevorzugten Ausführungsform wird ein Schalenkatalysator als erster Katalysator verwendet, welcher Aluminiumoxid als Trägermaterial und Palladium und Platin als Metalle umfasst und wobei der Katalysator eine Palladium-Konzentration von bis zu 2,0 Gew.-%, vorzugsweise von bis zu 1,0 Gew.-%, besonders bevorzugt von bis zu 0,5 Gew.-% und eine Platin-Konzentration von bis zu 1,0 Gew.-%, vorzugsweise von bis zu 0,5 Gew.-%, bevorzugt von bis zu 0,2 Gew.-% aufweist. Dabei sollte die Konzentration des Palladiums mindestens um den Faktor 2 höher sein als die des Platins.

Der erste Katalysator weist vorzugsweise eine spezifische Oberfläche von 50 bis 400 m²/g, bevorzugt von 100 bis 300 m²/g, besonders bevorzugt von 200 bis 300 m²/g auf, die mittels Gasadsorption gemäß DIN ISO 9277 (Stand: Stand: 2014-01) bestimmt werden kann.

Die Eintrittstemperatur des olefinhaltigen C4-Kohlenwasserstoffstroms in den oder die Reaktoren der ersten Reaktionszone liegt im ersten Schritt vorzugsweise im Bereich von 0 bis 180°C, vorzugsweise im Bereich von 60 bis 150°C und besonders bevorzugt im Bereich von 80 bis 130°C. Der Druck liegt im ersten Schritt vorzugsweise im Bereich von 2 bis 50 bar, vorzugsweise im Bereich von 6 bis 40 bar und besonders bevorzugt im Bereich von 10 bis 30 bar.

Die Hydrierung und/oder Umsetzung im ersten Schritt wird vorzugsweise als Flüssigphasenverfahren betrieben, das heißt, dass alle Komponenten in der ersten Reaktionszone, also dem mindestens einen Reaktor, in flüssiger Phase vorliegen oder flüssig in die erste Reaktionszone eingebracht werden. Geeignete Reaktoren für die Durchführung der Umsetzung im ersten Schritt sind Festbettreaktoren oder Rohrbündelreaktoren. Der Wasserstoff liegt in der flüssigen Phase vorzugsweise vollständig gelöst vor. Es ist klar, dass die Temperatur und der Druck dann so gewählt werden müssen, dass der Wasserstoff vollständig gelöst bleibt und sich vor und in der Reaktionszone keine Gasphase ausbildet. Die Zugabe von Wasserstoff zu dem olefinhaltigen C4-Kohlenwasserstoffstrom sollte demnach in fein verteilter Form und in solchen Mengen erfolgen, dass vor und in der Reaktionszone immer eine homogene Flüssigphase vorliegt. Es erfolgt keine Zugabe von Kohlenmonoxid. Die Hydrierung und/oder Umsetzung im ersten Schritt wird weiterhin vorzugsweise in nur einem Reaktor durchgeführt, d. h. die erste Reaktionszone besteht vorzugsweise aus nur einem Reaktor.

Nach der Hydrierung der mehrfach ungesättigten Kohlenwasserstoffe und/oder der Umsetzung der mehrfach ungesättigten Kohlenwasserstoffe mit den Mercaptanen und/oder Disulfiden im ersten Schritt ist eine Abtrennung der Umsetzungsprodukte im Rahmen einer Schwersiederabtrennung, die zwischen dem ersten und dem zweiten Schritt angeordnet ist, möglich. Die Abtrennung erfolgt vorzugsweise mittels Destillation. Dabei können die im C4-Kohlenwasserstoffstrom befindlichen Schwersieder wie C5-Kohlenwasserstoffe zumindest teilweise zusammen mit den Umsetzungsprodukten abgetrennt werden. Die Schwersieder, also Umsetzungsprodukte und C5-Kohlenwasserstoffe, werden über den Sumpf der Destillationskolonne abgezogen, der aufgereinigte C4-Kohlenwasserstoffstrom wird über Kopf der Destillationskolonne abgezogen und zum zweiten Schritt geleitet. Vor der Durchführung des zweiten Schrittes können weitere Verfahrensschritte durchgeführt werden, bei denen die mehrfach ungesättigten Kohlenwasserstoffe nicht stören, beispielsweise eine MTBE-Synthese (wodurch Isobuten abgetrennt werden kann) oder eine TBA-Synthese.

Eine bei der Schwersiederabtrennung vorzugsweise eingesetzte Destillationskolonne weist vorzugsweise 40 bis 150 theoretische Trennstufen, vorzugsweise 40 bis 100 theoretische Trennstufen und besonders bevorzugt 50 bis 80 theoretische Trennstufen auf. Das Rücklaufverhältnis kann, in Abhängigkeit von der vorhandenen Anzahl an Trennstufen, der Zusammensetzung des Zulaufes und der erforderlichen Reinheit von Kopf- und Sumpfprodukt, zwischen 0,5 und 5, besonders bevorzugt zwischen 1 und 2,5 betragen. Das Rücklaufverhältnis ist hierbei definiert als Massenstrom des Rücklaufs geteilt durch den Massenstrom des Kopfprodukte (Destillat).

Die Destillationskolonne für die Schwersiederabtrennung wird vorzugsweise mit einem Druck von 1 bis 20 bar (absolut), vorzugsweise von 5 bis 12 bar (absolut) betrieben. Die Kondensation kann, je nach vorhandenem Betriebsdruck, gegen Kühlsole, Kühlwasser oder Luft erfolgen. Das Sumpfprodukt, also die Schwersieder, kann thermisch verwertet werden oder als Einsatzstoff anderer Prozesse, beispielsweise in einer Synthesegasanlage, genutzt werden.

Nach Abtrennung der Schwersieder wird das C4-Kohlenwasserstoffgemisch, welches noch mehrfach ungesättigte Kohlenwasserstoffe enthält, zum zweiten Schritt des erfindungsgemäßen Verfahrens, also der Selektivhydrierung, geführt. In dem zweiten Schritt wird ebenfalls Wasserstoff zugesetzt, diesmal jedoch nicht im Unterschuss wie beim ersten Schritt, sondern mindestens äquimolar zur im Zulauf des zweiten Schrittes vorhandenen molaren Menge an mehrfach ungesättigten Kohlenwasserstoffen. Das molare Verhältnis von Wasserstoff zu den mehrfach ungesättigten Kohlenwasserstoffen liegt insbesondere im Bereich von 2 : 1 und 1 : 1 (Wasserstoff: mehrfach ungesättigte Kohlenwasserstoffe), vorzugsweise im Bereich von 1,5 : 1 und 1 : 1, besonders bevorzugt im Bereich von 1,2 : 1 und 1 : 1. Der Wasserstoff kann dabei separat zum einströmenden C4-Kohlenwasserstoffstrom in den oder die Reaktoren der zweiten Reaktionszone eindosiert werden oder dem einströmenden C4-Kohlenwasserstoffstrom vorher hinzugefügt werden.

Dem C4-Kohlenwasserstoffstrom wird im zweiten Schritt zusätzlich Kohlenmonoxid zugegeben. Der Gehalt an Kohlenmonoxid im C4-Kohlenwasserstoffstrom im Zulauf der zweiten Reaktionszone beträgt vorzugsweise zwischen 0,05 und 20 ppm Kohlenmonoxid, besonders bevorzugt zwischen 0,5 und 5 ppm, jeweils bezogen auf die Gesamtmenge des C4-Kohlenwasserstoffstroms. Das Kohlenmonoxid kann dabei separat zum einströmenden C4-Kohlenwasserstoffstrom in die zweite Reaktionszone eindosiert werden oder dem einströmenden C4-Kohlenwasserstoffstrom vorher hinzugefügt werden.

Als zweiter Katalysator werden im zweiten Schritt des erfindungsgemäßen Verfahrens heterogene Katalysatoren eingesetzt, die ein einziges Metall der 10. Gruppe des Periodensystems der Elemente enthalten, vorzugsweise Palladium. Das Metall kann aufgrund von Kalzination partiell in Form seines Oxids vorliegen. Vorzugsweise liegt das Palladium auf einem Trägermaterial vor. Das Trägermaterial ist ausgewählt aus der Gruppe, bestehend aus Aluminiumoxid, Kieselgel und Aktivkohle, wobei bevorzugt Aluminiumoxid als Trägermaterial eingesetzt wird. Die Konzentration des Metalls im zweiten Katalysator kann 0,01 bis 3 Gew.-%, vorzugsweise 0,1 bis 1 Gew.-%, besonders bevorzugt 0,3 bis 0,5 Gew.-%, jeweils bezogen auf die Gesamtmenge des Katalysators, betragen. Der zweite Katalysator ist erfindungsgemäß unterschiedlich zum ersten Katalysator.

Der zweite Katalysator weist vorzugsweise eine spezifische Oberfläche von 50 bis 400 m²/g, bevorzugt von 100 bis 300 m²/g, besonders bevorzugt von 200 bis 300 m²/g auf, die mittels Gasadsorption gemäß DIN ISO 9277 (Stand: Stand: 2014-01) bestimmt werden kann.

Die Eintrittstemperatur des C4-Kohlenwasserstoffstroms im Zulauf zur zweiten Reaktionszone liegt üblicherweise im Bereich von 0 bis 100 °C, vorzugsweise im Bereich von 20 bis 80 °C, besonders bevorzugt im Bereich von 30 bis 60 °C. Der Druck liegt üblicherweise im Bereich von 2 bis 50 bar, vorzugsweise im Bereich von 6 bis 30 bar, besonders bevorzugt im Bereich von 10 bis 25 bar.

Die Selektivhydrierung im zweiten Schritt wird vorzugsweise als Flüssigphasenverfahren betrieben, das heißt, dass alle Komponenten in der zweiten Reaktionszone in flüssiger Phase vorliegen oder flüssig in die erste Reaktionszone eingebracht werden. Geeignete Reaktoren für die Durchführung der Selektivhydrierung sind Festbettreaktoren oder Rohrbündelreaktoren. Der Wasserstoff liegt ebenso wie das Kohlenmonoxid in der flüssigen Phase vorzugsweise vollständig gelöst vor. Es ist klar, dass die Temperatur und der Druck dann so gewählt werden müssen, dass der Wasserstoff und das Kohlenmonoxid vollständig gelöst bleiben und sich vor und in der Reaktionszone keine Gasphase ausbildet.

Die Selektivhydrierung im zweiten Schritt kann einstufig, das heißt unter Verwendung eines einzigen Reaktors in der zweiten Reaktionszone betrieben werden. Bevorzugt wird die Selektivhydrierung mehrstufig, also unter Verwendung von mindestens zwei parallel oder in Reihe geschalteter Reaktoren in der zweiten Reaktionszone, betrieben, d. h. die zweite Reaktionszone besteht aus mindestens zwei parallel oder in Reihe geschalteter Reaktoren. Die Reaktoren liegen dabei vorzugsweise in Reihe geschaltet in der zweiten Reaktionszone vor. Dabei wird der Wasserstoff in jeden der in Reihe geschalteten Reaktoren in der zweiten Reaktionszone zusammen mit dem C4-Kohlenwasserstoffstrom eingespeist, während das Kohlenmonoxid nur in den ersten der in Reihe geschalteten Reaktoren der zweiten Reaktionszone zugegeben wird, nicht jedoch in den zweiten oder nachfolgende Reaktoren.

In einer anderen Ausführungsform des erfindungsgemäßen Verfahrens besteht die zweite Reaktionszone aus mindestens drei Reaktoren. Dabei können die ersten beiden parallel geschaltet vorliegen und gemeinsam in Reihe zum dritten Reaktor geschaltet sein. In die ersten beiden Reaktoren, die parallel geschaltet vorliegen, werden vorzugsweise Wasserstoff und Kohlenmonoxid zudosiert, während im dritten Reaktor nur Wasserstoff zugegeben wird.

### Beispiel 1:

In einen ersten Reaktor, der mit 600 ml eines Hydrierkatalysators (0,5 Gew.-% Pd, 0,2 Gew.-% Pt auf Al₂O₃) gefüllt und auf 90°C temperiert ist, wird mit 4 kg/h ein bei 22 bar Druck verflüssigtes C4-Gemisch bestehend aus 0,7 Gew.-% 1,3-Butadien, 19,9 Gew.-% 1-Buten, 38,0 Gew.-% 2-Butenen, 29,3 Gew.-% Isobuten und 11,3 Gew.-% Butanen, sowie 0,8 Gew.-% C5-Kohlenwasserstoffen gefahren. Der Strom enthält etwa 4 Gew.-ppm Dimethyldisulfid, etwa 3 Gew.-ppm Ethanthiol und etwa 0,5 Gew.-ppm Methanthiol.

In dieses C4-Gemisch wird ein H₂-Strom von 9,3 NI/h hinzudosiert. Das im eingesetzten C4-Gemisch vorhandene Butadien wird zu etwa 73 % umgesetzt. Damit ergibt sich eine Zusammensetzung des Austrages des ersten Reaktors von 0,2 Gew.-% Butadien, 20,2 Gew.-% 1-Buten, 38,2 Gew.-% 2-Buten, 29,3 Gew.-% Isobuten und 11,3 Gew.-% Butane, sowie 0,8 Gew.-% C5-Kohlenwasserstoffe. Mercaptane und Disulfide waren im Strom nicht mehr nachweisbar.

Nach einer Schwersiederabtrennung und einer weitgehenden Abtrennung des Isobutens durch eine MTBE-Synthese setzt sich der Strom wie folgt zusammen: 0,3 Gew.-% Butadien, 28,6 Gew.-% 1-Buten, 54,0 Gew.-% 2-Buten, 1,1 Gew.-% Isobuten und 16,0 Gew.-% n-Butan. Dieser Strom wird bei 12 bar Druck verflüssigt und mit 4 kg/h in einen zweiten Reaktor, der mit 600 ml eines Hydrierkatalysators (0,5 Gew.-% Pd auf Al₂O₃) gefüllt und auf 40°C temperiert ist, gefahren. In dieses C4-Gemisch wird ein H₂-Strom von 3,5 NI/h hinzudosiert. Zusätzlich werden 3 Nml/h CO zudosiert. Nach Durchfahren des zweiten Reaktors ist im Strom kein Butadien mehr nachweisbar.

## Patentansprüche

1. Verfahren zur Entfernung von mehrfach ungesättigten Kohlenwasserstoffen aus olefinhaltigen C4-Kohlenwasserstoffströmen, welche neben C4-Kohlenwasserstoffen auch C5-Kohlenwasserstoffe im Bereich zwischen 0,01 und 5 Gew.-% und Mercaptane und/oder Disulfide im Bereich zwischen 0,01 und 200 Gew.-ppm enthalten, wobei
in einem ersten Schritt zumindest ein Teil der mehrfach ungesättigte Kohlenwasserstoffe aus dem olefinhaltigen C4-Kohlenwasserstoffstrom in einer ersten Reaktionszone, die aus einem oder mehreren parallel und/oder in Reihe geschalteten Reaktoren besteht hydriert, und/oder mit den Mercaptanen und/oder Disulfiden aus dem olefinhaltigen C4-Kohlenwasserstoffstrom umgesetzt werden, wobei der erste Schritt unter Verwendung eines ersten Katalysators, der mindestens zwei Metalle der 10. Gruppe des Periodensystems der Elemente, vorzugsweise Palladium und Platin, enthält, und in Anwesenheit von Wasserstoff und in Abwesenheit von Kohlenmonoxid durchgeführt wird und wobei in dem ersten Schritt maximal 80 Mol-% der vorhandenen mehrfach ungesättigten Kohlenwasserstoffe aus dem C4-Kohlenwasserstoffstrom hydriert und/oder umgesetzt werden, wobei das molare Verhältnis von Wasserstoff zu mehrfach ungesättigten Kohlenwasserstoffen zwischen 0,01 und 0,8 beträgt,
in einem zweiten Schritt die im ersten Schritt nicht umgesetzten mehrfach ungesättigten Kohlenwasserstoffe in dem olefinhaltigen C4-Kohlenwasserstoffstrom in einer zweiten Reaktionszone, die aus einem oder mehreren parallel und/oder in Reihe geschalteten Reaktoren besteht, unter Verwendung eines zweiten Katalysators, der ein einziges Metall der 10. Gruppe des Periodensystems der Elemente, vorzugsweise Palladium, enthält, in Anwesenheit von Wasserstoff und Kohlenmonoxid selektiv hydriert werden,
wobei zwischen dem ersten und dem zweiten Schritt mindestens eine Schwersiederabtrennung erfolgt, und
wobei der erste und der zweite Katalysator unterschiedlich voneinander sind.

2. Verfahren nach Anspruch 1, wobei im ersten Schritt das molare Verhältnis von Wasserstoff zu mehrfach ungesättigten Kohlenwasserstoffen zwischen 0,1 und 0,5 beträgt.

3. Verfahren nach Anspruch 1 oder 2, wobei die Eintrittstemperatur des C4-Kohlenwasserstoffstroms im Zulauf der ersten Reaktionszone, die aus einem oder mehreren parallel und/oder in Reihe geschalteten Reaktoren besteht, zwischen 0°C und 180°C, vorzugsweise zwischen 60°C und 150°C, besonders bevorzugt zwischen 80°C und 130°C beträgt.

4. Verfahren nach einem der Ansprüche 1 bis 3, wobei der erste Schritt ausschließlich in der flüssigen Phase durchgeführt wird und der vorhandene Wasserstoff vollständig in der flüssigen Phase gelöst ist.

5. Verfahren nach einem der Ansprüche 1 bis 4, wobei im zweiten Schritt der Gehalt an Kohlenmonoxid im C4-Kohlenwasserstoffstrom im Zulauf der zweiten Reaktionszone, die aus einem oder mehreren parallel und/oder in Reihe geschalteten Reaktoren besteht, 0,05 bis 20 ppm beträgt, bezogen auf die Gesamtmenge des Kohlenwasserstoffstroms.

6. Verfahren nach einem der Ansprüche 1 bis 5, wobei die Eintrittstemperatur des C4-Kohlenwasserstoffstroms im Zulauf der zweiten Reaktionszone, die aus einem oder mehreren parallel und/oder in Reihe geschalteten Reaktoren besteht, zwischen 0°C und 100°C, vorzugsweise zwischen 20 und 80 °C, besonders bevorzugt zwischen 30 und 60 °C beträgt.

7. Verfahren nach einem der Ansprüche 1 bis 6, wobei der Druck in der zweiten Reaktionszone, die aus einem oder mehreren parallel und/oder in Reihe geschalteten Reaktoren besteht, im Bereich von 2 bis 50 bar, vorzugsweise im Bereich von 6 bis 30 bar, besonders bevorzugt im Bereich von 10 bis 25 bar liegt.

8. Verfahren nach einem der Ansprüche 1 bis 7, wobei der zweite Schritt ausschließlich in der flüssigen Phase durchgeführt wird.

9. Verfahren nach einem der Ansprüche 1 bis 8, wobei die erste Reaktionszone aus einem einzigen Reaktor besteht.

10. Verfahren nach einem der Ansprüche 1 bis 9, wobei die zweite Reaktionszone aus mindestens zwei parallel oder in Reihe geschalteter Reaktoren besteht.

## Claims

1. Process for removing polyunsaturated hydrocarbons from olefin-containing C4 hydrocarbon streams that, in addition to C4 hydrocarbons, also contain C5 hydrocarbons in the range between 0.01% and 5% by weight and mercaptans and/or disulfides in the range between 0.01% and 200 ppm by weight, wherein
in a first step, at least some of the polyunsaturated hydrocarbons from the olefin-containing C4 hydrocarbon stream are hydrogenated and/or reacted with the mercaptans and/or disulfides from the olefin-containing C4 hydrocarbon stream in a first reaction zone, which consists of one or more reactors connected in parallel and/or in series, wherein the first step is carried out using a first catalyst containing at least two metals of group 10 of the periodic table of the elements, preferably palladium and platinum, and in the presence of hydrogen and in the absence of carbon monoxide and wherein not more than 80 mol% of the available polyunsaturated hydrocarbons from the C4 hydrocarbon stream is hydrogenated and/or reacted in the first step, wherein the molar ratio of hydrogen to polyunsaturated hydrocarbons is between 0.01 and 0.8, in a second step, the polyunsaturated hydrocarbons in the olefin-containing C4 hydrocarbon stream that did not react in the first step are selectively hydrogenated using a second catalyst containing a single metal of group 10 of the periodic table of the elements, preferably palladium, in the presence of hydrogen and carbon monoxide in a second reaction zone, which consists of one or more reactors connected in parallel and/or in series,
wherein at least one removal of high boilers takes place between the first step and the second step, and wherein the first and second catalysts are different from one another.

2. Process according to Claim 1, wherein the molar ratio of hydrogen to polyunsaturated hydrocarbons in the first step is between 0.1 and 0.5.

3. Process according to Claim 1 or 2, wherein the inlet temperature of the C4 hydrocarbon stream in the inflow of the first reaction zone, which consists of one or more reactors connected in parallel and/or in series, is between 0°C and 180°C, preferably between 60°C and 150°C, more preferably between 80°C and 130°C.

4. Process according to any of Claims 1 to 3, wherein the first step is carried out exclusively in the liquid phase and the hydrogen present has been dissolved completely in the liquid phase.

5. Process according to any of Claims 1 to 4, wherein, in the second step, the content of carbon monoxide in the C4 hydrocarbon stream in the inflow of the second reaction zone, which consists of one or more reactors connected in parallel and/or in series, is 0.05 to 20 ppm based on the total amount of the hydrocarbon stream.

6. Process according to any of Claims 1 to 5, wherein the inlet temperature of the C4 hydrocarbon stream in the inflow of the second reaction zone, which consists of one or more reactors connected in parallel and/or in series, is between 0°C and 100°C, preferably between 20°C and 80°C, more preferably between 30°C and 60°C.

7. Process according to any of Claims 1 to 6, wherein the pressure in the second reaction zone, which consists of one or more reactors connected in parallel and/or in series, is in the range from 2 to 50 bar, preferably in the range from 6 to 30 bar, more preferably in the range from 10 to 25 bar.

8. Process according to any of Claims 1 to 7, wherein the second step is carried out exclusively in the liquid phase.

9. Process according to any of Claims 1 to 8, wherein the first reaction zone consists of a single reactor.

10. Process according to any of Claims 1 to 9, wherein the second reaction zone consists of at least two reactors connected in parallel or in series.

## Revendications

1. Procédé d'élimination d'hydrocarbures polyinsaturés à partir de courants d'hydrocarbures en C4 contenant des oléfines, qui, outre des hydrocarbures en C4 contiennent aussi des hydrocarbures en C5 dans la plage de 0,01 à 5 % en poids et des mercaptans et/ou des disulfures dans la plage de 0,01 à 200 ppm en poids, dans lequel
dans une première étape, au moins une partie des hydrocarbures polyinsaturés provenant du courant d'hydrocarbures en C4 contenant des oléfines sont hydrogénés dans une première zone de réaction qui est constituée d'un ou plusieurs réacteurs montés en parallèle et/ou en série, et/ou sont mis à réagir avec les mercaptans et/ou les disulfures provenant du courant d'hydrocarbures en C4, la première étape étant mise en œuvre par utilisation d'un premier catalyseur, qui contient au moins deux métaux du 10e groupe du tableau périodique des éléments, de préférence du palladium et du platine, et en présence d'hydrogène et en l'absence de monoxyde de carbone, et dans la première étape au maximum 80 % en moles des hydrocarbures polyinsaturés présents provenant du courant d'hydrocarbures en C4 sont hydrogénés et/ou mis à réagir, le rapport en moles de l'hydrogène aux hydrocarbures polyinsaturés étant compris entre 0,01 et 0, 8,
dans une deuxième étape, les hydrocarbures polyinsaturés qui n'ont pas réagi dans la première étape et se trouvant dans le courant d'hydrocarbures en C4 contenant des oléfines sont, dans une seconde zone de réaction, qui est constituée d'un ou plusieurs réacteurs montés en parallèle et/ou en série, sélectivement hydrogénés par utilisation d'un second catalyseur, qui contient un métal unique du 10e groupe du tableau périodique des éléments, de préférence du palladium, en présence d'hydrogène et de monoxyde de carbone,
au moins une séparation des substances à haut point d'ébullition étant réalisée entre la première et la seconde étapes, et
le premier et le second catalyseur étant différents l'un de l'autre.

2. Procédé selon la revendication 1, dans lequel, dans la première étape, le rapport en moles de l'hydrogène aux hydrocarbures polyinsaturés est compris entre 0,1 et 0,5.

3. Procédé selon la revendication 1 ou 2, dans lequel la température d'entrée du courant d'hydrocarbures en C4 à l'entrée de la première zone de réaction, qui est constituée d'un ou plusieurs réacteurs montés en parallèle et/ou en série, est comprise entre 0 °C et 180 °C, de préférence entre 60 °C et 150 °C, d'une manière particulièrement préférée entre 80 °C et 130 °C.

4. Procédé selon l'une des revendications 1 à 3, dans lequel la première étape est mise en œuvre exclusivement en phase liquide, et l'hydrogène présent est entièrement dissous dans la phase liquide.

5. Procédé selon l'une des revendications 1 à 4, dans lequel dans la deuxième étape, la teneur du courant d'hydrocarbures en C4 en monoxyde de carbone à l'entrée de la seconde zone de réaction, qui est constituée d'un ou plusieurs réacteurs montés en parallèle et/ou en série, est de 0,05 à 20 ppm, par rapport à la quantité totale du courant d'hydrocarbures.

6. Procédé selon l'une des revendications 1 à 5, dans lequel la température d'entrée du courant d'hydrocarbures en C4 à l'entrée de la seconde zone de réaction, qui est constituée d'un ou plusieurs réacteurs montés en parallèle et/ou en série, est comprise entre 0 °C et 100 °C, de préférence entre 20 et 80 °C, d'une manière particulièrement préférée entre 30 et 60 °C.

7. Procédé selon l'une des revendications 1 à 6, dans lequel la pression dans la seconde zone de réaction, qui est constituée d'un ou plusieurs réacteurs montés en parallèle et/ou en série, est comprise dans la plage de 2 à 50 bar, de préférence dans la plage de 6 à 30 bar et d'une manière particulièrement préférée dans la plage de 10 à 25 bar.

8. Procédé selon l'une des revendications 1 à 7, dans lequel la seconde étape est mise en œuvre exclusivement en phase liquide.

9. Procédé selon l'une des revendications 1 à 8, dans lequel la première zone de réaction est constituée d'un réacteur unique.

10. Procédé selon l'une des revendications 1 à 9, dans lequel la seconde zone de réaction est constituée d'au moins deux réacteurs montés en parallèle ou en série.
